# EUROPEAN PATENT APPLICATION

(11) **EP 3 246 085 A1**
(43) Date of publication of application: **22.11.2017**
(21) Application number: 15877916.5
(22) Date of filing: 09.10.2015
(51) Int. Cl.: B01D 71/68, B01D 69/02, B01D 69/08, B01D 71/44, C08L 81/06

(54) **POROUS HOLLOW FIBER MEMBRANE**

(30) Priority: 16.01.2015 JP 2015007073
(71) Applicant: Asahi Kasei Medical Co., Ltd., Chiyoda-ku Tokyo 101-8101 (JP)
(72) Inventor: KAYAMA, Yuzo, Tokyo 101-8101 (JP); KOMURO, Masayasu, Tokyo 101-8101 (JP)
(74) Representative: dompatent von Kreisler Selting Werner - Partnerschaft von Patent- und Rechtsanwälten mbB
(86) International application number: PCT/JP2015/078848
(87) International publication number: WO 2016/113964

(57) **Abstract**

The present invention provides a porous hollow fiber membrane including a polysulfone-based polymer and a hydrophilic polymer, and having a dense layer in a section from an outer surface portion to a center region of a membrane thickness, a thickness of the dense layer being 10 to 30 µm, and a ratio of a pore having a pore size of more than 50 nm and a ratio of a pore having a pore size of 10 nm or smaller in the dense layer being 25 to 40% and 20% or less, respectively.

## Description

### Technical Field

The present invention relates to a porous hollow fiber membrane.

### Background Art

A hollow fiber membrane is widely utilized in industrial applications such as microfiltration and ultrafiltration, and medical applications such as hemodiafiltration. In recent years, use of a hollow fiber membrane in an application to virus removal in purification steps of plasma derivatives and bio-pharmaceuticals has been spread. A hollow fiber membrane for use in an application to virus removal is demanded to satisfy not only virus removability, but also suppressing the lowering of flux with time during filtration to provide productivity by enhanced protein permeability.

A virus removal or inactivation method includes a heating treatment, an optical treatment and a treatment with chemicals. A membrane filtration method, which is effective for all viruses regardless of thermal and chemical properties of viruses, has attracted attention in terms of, for example, protein denaturation, inactivation efficiency, and/or contamination of chemicals.

With respect to viruses to be removed or inactivated, the smallest virus includes parvovirus having a diameter of 18 to 24 nm and also poliovirus having a diameter of 25 to 30 nm, and a relatively larger virus includes HIV virus having a diameter of 80 to 100 nm. In recent years, there has been a growing need for removal of small viruses such as parvovirus.

A hollow fiber membrane for use in purification steps of plasma derivatives and bio-pharmaceuticals is demanded to achieve efficient protein recovery such as 5-nm albumin and 10-nm globulin in terms of productivity. An ultrafiltration membrane and a hemodialysis membrane having a pore size of about several nm, and furthermore a reverse osmosis membrane having a smaller pore size are not suitable as a membrane for protein treatment because pores thereof are blocked by protein during filtration. In particular, when removal of small viruses such as parvovirus is intended, it is difficult to satisfy both of virus removability and efficient protein recovery.

Patent Literature 1 discloses a virus removal membrane comprising a hollow fiber membrane formed from a blend of a polysulfone-based polymer and polyvinylpyrrolidone (PVP), wherein when a 0.5% immunoglobulin solution is subjected to filtration at a constant pressure of 1.0 bar for 60 minutes in dead-end filtration mode, its filtration time and integrated amount of recovered filtrate are substantially in a linear relation.

Patent Literature 2 discloses a virus removal membrane comprising a hollow fiber membrane formed from a blend of a polysulfone-based polymer and a copolymer of vinylpyrrolidone and vinyl acetate, wherein the hollow fiber membrane is coated with a polysaccharide derivative to thereby inhibit the copolymer of vinylpyrrolidone and vinyl acetate from being eluted from the hollow fiber membrane. Patent Literature 2 also discloses the amount of the filtrate until the filtration pressure reaches 3 bar in filtration of an immunoglobulin solution at a constant filtration rate of 120 L/m²·hr.

Patent Literature 3 discloses a hollow fiber membrane obtained by dissolving a polysulfone-based polymer and polyvinylpyrrolidone and spinning the resultant, and also discloses that the hollow fiber membrane has a dense-coarse-dense structure from the vicinity of the inner periphery towards the vicinity of the outer periphery.

### Citation List

### Patent Literature

Patent Literature 1: International Publication No. 2011/111679
Patent Literature 2: Japanese Patent No. 5403444
Patent Literature 3: Japanese Patent Laid-Open No. 2013-71100

### Summary of Invention

### Technical Problem

Patent Literature 1 describes, as an index of clogging, a linear relationship in a graph with the filtration time represented on the horizontal axis and the integrated amount of a filtrate recovered represented on the vertical axis. However, when the graph is created for the hollow fiber membrane disclosed in Patent Literature 1, no line through the origin is provided. Conversely, such a relationship is far from a linear relationship even when subjected to linear regression through the origin, and only a membrane is disclosed in which clogging substantially occurs even when a 0.5% immunoglobulin solution is subjected to filtration at a constant pressure of 1.0 bar for 60 minutes in dead-end filtration mode. In addition, while Patent Literature 1 defines that the hollow fiber membrane disclosed therein has a homogeneous center region, it has substantially the same dense-coarse-dense membrane structure as that in Patent Literature 3, and has a dense layer serving as a virus capture region in each of the vicinity of the outer surface and the vicinity of the inner surface.

In the hollow fiber membrane disclosed in Patent Literature 2, the filtration pressure increases to 3 bar when filtration at a constant rate is performed, and therefore it is meant that clogging substantially occurs. In addition, Patent Literature 2 discloses no method for suppressing the lowering of the flux with time.

A problem to be solved by the present invention is to provide a hollow fiber membrane which exhibits sufficient performance for removing a virus and the like contaminated in a solution, and suppresses the lowering of the flux with time during filtration of a protein solution to provide excellent protein permeability.

### Solution to Problem

The present inventors have made intensive studies in order to solve the above problem, and as a result, have found that the above problem can be solved by a porous hollow fiber membrane having a specified dense layer, thereby completing the present invention.

That is, the present invention is as follows.
(1) A porous hollow fiber membrane including a polysulfone-based polymer and a hydrophilic polymer, and having a dense layer in a section from an outer surface portion to a center region of a membrane thickness, a thickness of the dense layer being 10 to 30 µm, and a ratio of a pore having a pore size of more than 50 nm and a ratio of a pore having a pore size of 10 nm or smaller in the dense layer being 25 to 40% and 20% or less, respectively.
(2) The porous hollow fiber membrane according to (1), wherein the polysulfone-based polymer is a polyethersulfone.
(3) The porous hollow fiber membrane according to (1) or (2), wherein the hydrophilic polymer is a copolymer of vinylpyrrolidone and vinyl acetate.
(4) The porous hollow fiber membrane according to any of (1) to (3), wherein a pure water permeation rate is 40 to 180 L/(hr·m²·bar).
(5) The porous hollow fiber membrane according to any of (1) to (4), wherein, when 1.5% by mass immunoglobulin is filtered at a constant pressure of 2.0 bar from an inner surface of a hollow fiber to an outer surface thereof, a ratio (F₆₀/F₁₀) of immunoglobulin flux F₆₀ from a point of a lapse of 50 minutes to a point of a lapse of 60 minutes after the start of filtration to immunoglobulin flux F₁₀ from the start of filtration to a point of a lapse of 10 minutes after the start of filtration is 0.60 or more.
(6) The porous hollow fiber membrane according to any of (1) to (5), for use in removing a virus contaminated in a protein solution and recovery of protein.

### Advantageous Effects of Invention

The present invention provides a hollow fiber membrane which exhibits sufficient performance for removing a virus and the like contaminated in a solution, and suppresses the lowering of the flux with time during filtration of a protein solution to provide excellent protein permeability.

### Brief Description of Drawing

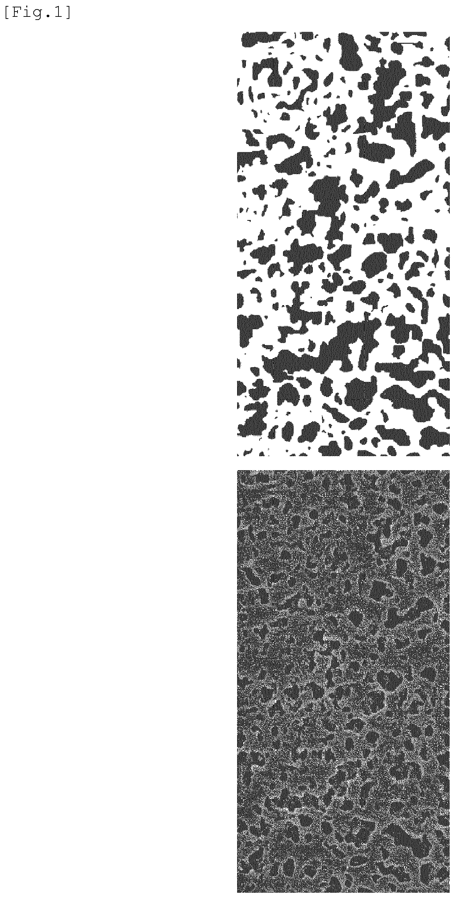

[Fig.1] Fig. 1 illustrates the results of binarization of an image observed by a scanning electron microscope to a pore portion and a solid portion. A black portion corresponds to the pore portion, and a white portion corresponds to the solid portion.

### Description of Embodiments

Hereinafter, an embodiment for carrying out the present invention (hereinafter, referred to as "the present embodiment".) is described in detail. The present invention is not limited to the following embodiment, and can be carried out with being variously modified within the gist thereof.

A porous hollow fiber membrane of the present embodiment includes
a polysulfone-based polymer and a hydrophilic polymer, and
has a dense layer in a section from an outer surface portion to the center region of the membrane thickness, the thickness of the dense layer is 10 to 30 µm, and the ratio of a pore having a pore size of more than 50 nm and the ratio of a pore having a pore size of 10 nm or smaller in the dense layer are 25 to 40% and 20% or less, respectively.

The porous hollow fiber membrane of the present embodiment includes a polysulfone-based polymer and a hydrophilic polymer.

The polysulfone-based polymer is a polysulfone (PSf) having a repeating unit represented by the following formula 1 or a polyethersulfone (PES) having a repeating unit represented by the following formula 2, and is preferably PES.

The polysulfone-based polymer may include a substituent such as a functional group or an alkyl group in any structure represented by the following formula 1 or 2, and a hydrogen atom of a hydrocarbon skeleton may be substituted with other atom such as halogen, or a substituent.

The polysulfone-based polymers may be used singly or in the form of a mixture of two or more.

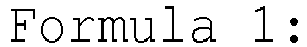

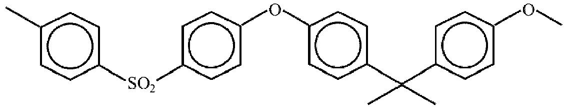

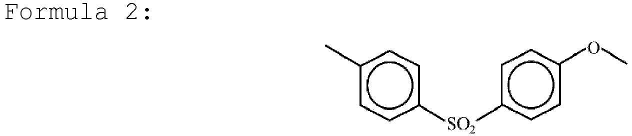

The hydrophilic polymer is not particularly limited as long as it is compatible with the polysulfone-based polymer in a good solvent, and is preferably a copolymer of vinylpyrrolidone and vinyl acetate.

Specific examples of the hydrophilic polymer include LUVISKOL (trade name) VA64 and VA73 commercially available from BASF SE.

The hydrophilic polymers may be used singly or in the form of a mixture of two or more.

A membrane which exhibits sufficient virus removability and is excellent in protein permeability is demanded in an application to protein solution treatment.

A membrane having the following characteristics can be a useful membrane in an application to protein solution treatment: (1) the membrane can be operated at high filtration pressure; and (2) the membrane has a large number of pores through which a monomer of protein as a useful component can permeate and which can capture foreign substances such as an aggregate of protein, and viruses.

The characteristic (1), that is, the operation at high filtration pressure, can be realized by using, as a base material, a polysulfone-based polymer having pressure resistance.

When the membrane has the characteristic (2), that is, the membrane has a large number of pores through which a monomer of protein as a useful component can permeate and which can capture foreign substances such as an aggregate of protein, and viruses, the ratio of pores blocked can be reduced in the entire membrane. As a result, the lowering of flux with time during filtration of a protein solution is suppressed to enable efficient protein recovery.

An increase in the number of pores through which a monomer of protein as a useful component permeate and which can capture foreign substances such as an aggregate of protein, and viruses, can be achieved by increasing a region where a large number of such pores are present.

The thickness of the dense layer can be increased to thereby increase the number of pores through which a monomer of protein as a useful component permeate and which can capture foreign substances such as an aggregate of protein, and viruses. However, when the thickness of the dense layer is too much increased, the flux itself decreases.

The present inventors have made studies and thus have found that when a porous hollow fiber membrane include a dense layer with a thickness of 10 to 30 µm in a section from an outer surface portion to the center region of the membrane thickness, the flux can be controlled in a suitable range to suppress the lowering of flux due to clogging during filtration.

In the present embodiment, the outer surface portion refers to a section from an outer surface, namely, a membrane surface opposite to the hollow portion of the hollow fiber to 10% of the membrane thickness in the membrane thickness direction, and the center region of the membrane thickness refers to a section from 10% to 90% of the membrane thickness from the outer surface. In the present embodiment, the porous hollow fiber membrane has one dense layer which is continuous in the thickness direction without any rapture.

As long as the thickness of the dense layer is 10 to 30 µm, the membrane thickness is not particularly limited and is preferably 30 to 80 µm, more preferably 40 to 80 µm.

The present inventors have made intensive studies about a membrane structure which suppresses the lowering of flux and maintains virus removability, and as a result, have found that, preferably, in addition to providing a dense layer having a specified thickness, the structure of the dense layer is precisely designed.

Specifically, a large pore which is not blocked by foreign substances and viruses is allowed to be present in the dense layer. It has been found that, even if a small pore is blocked by foreign substances and viruses, a large pore can be present to ensure a flow pass of a solution filtered in the dense layer, thereby suppressing the lowering of flux.

A simple increase in average pore size in the dense layer can suppress the lowering of flux with time, but causes virus removability to be degraded. When the ratio of a pore of more than 50 nm in the dense layer is 25 to 40%, high protein permeability can be realized while virus removability is ensured.

In addition, it is considered that if a monomer of protein is captured in the dense layer, a flux decreases by clogging to reduce recovery efficiency of protein. The size of immunoglobulin, which is a main subject to be filtered, is about 10 nm, and therefore no pore of 10 nm or smaller is preferably present in the dense layer. It is difficult due to the principle of membrane formation to completely eliminate any pore of 10 nm or smaller. In the present embodiment, when the ratio of a pore of 10 nm or smaller in the dense layer is 20% or less, it is possible to suppress the lowering of flux due to clogging of a small pore.

In the present embodiment, the ratio of a pore having a pore size of more than 50 nm and the ratio of a pore having a pore size of 10 nm or smaller in the dense layer are 25 to 40% and 20% or less, respectively. The ratio of a pore having a pore size of more than 50 nm and the ratio of a pore having a pore size of 10 nm or smaller in the dense layer may be 30 to 40% and 20% or less, respectively; the ratio of a pore having a pore size of more than 50 nm and the ratio of a pore having a pore size of 10 nm or smaller in the dense layer may be 25 to 40% and 15% or less, respectively; and the ratio of a pore having a pore size of more than 50 nm and the ratio of a pore having a pore size of 10 nm or smaller in the dense layer is preferably 30 to 40% and 15% or less, respectively.

In the present embodiment, the hollow fiber membrane is formed by a wet phase separation method in order to allow a large pore to be present in the dense layer.

In membrane formation by a wet phase separation method, the pore size distribution in the dense layer is controlled by the diffusion rates of the solvent/non-solvent. A certain time is taken for diffusion of the solvent/non-solvent, and microlevel and macrolevel concentration distributions are generated in the membrane. Therefore the pore size distribution can be naturally made broader to allow a large pore to be present in the dense layer.

The ratios of a small pore and a large pore in the dense layer are controlled into the predetermined ranges in the present embodiment by a method described below.

As the pure water permeation rate is higher, the protein solution filtration rate is higher. Therefore, the pure water permeation rate serves as an index of the protein solution permeation rate.

The pure water permeation rate of the porous hollow fiber membrane in the present embodiment is preferably 40 to 180 L/(hr·m²·bar), more preferably 70 to 180 L/(hr·m²·bar).

When a pure water permeation rate is 40 L/(hr·m²·bar) or more, the filtration time is not so long, to recover protein at a high efficiency. When a pure water permeation rate is 180 L/(hr·m²·bar) or less, the pore size is suitable in terms of virus removability.

In the present embodiment, the pure water permeation rate can be measured by a method described in Examples.

In the present embodiment, the dense layer of the porous hollow fiber membrane can be identified by photographing the cross section of the hollow fiber by a scanning electron microscope (SEM).

For example, the acceleration voltage is set to 1 kV, the photographing magnification is set to 50,000, and the visual field is horizontally set at an arbitrary portion of the cross section of the hollow fiber in the membrane thickness direction. After photographing at one visual field, the visual field to be photographed is horizontally moved in the membrane thickness direction, and the next visual field is photographed. Such photographing operations are repeated to take the photograph of the cross section of the membrane without any space, and the photographs obtained are combined to thereby provide one photograph of the cross section of the membrane. With respect to the photograph of the cross section, the average pore size is calculated in an area of (2 µm in the circumferential direction of the membrane) × (1 µm from the outer surface towards the inner surface).

The calculation method of the average pore size corresponds to calculation by a method using image analysis. Specifically, the pore portion and the solid portion are subjected to binarization processing by use of Image-pro plus manufactured by MediaCybernetics Inc. The pore portion and the solid portion are distinguished based on the brightness, and a portion which cannot be distinguished and a noise are corrected by a free hand tool. An edge section that forms a contour of a pore portion, and a porous structure observed at the back of the pore portion are distinguished as the pore portion. After binarization processing, the area value of one pore is assumed to be the area of a true circle and the pore diameter is calculated therefrom. Such operations are performed with respect to each pore, and the average pore size is sequentially calculated with respect to each area of 2 µm × 1 µm. Here, a pore portion cut at the end of the visual field is also counted as such an area. A visual field where the average pore size is 50 nm or smaller is defined as the dense layer, and a visual field where the average pore size is more than 50 nm is defined as a coarse layer. Specific results obtained by binarization of the SEM image are illustrated in Fig. 1.

The ratio of a pore in the dense layer may be calculated from the ratio of the number of pores having a pore size of more than 50 nm to the total number of pores in all visual fields of the dense layer, or may be calculated by determining the ratio of the number of pores to the total number of pores in one visual field of the dense layer, as the average in each visual field.

The ratio of a pore having a pore size of more than 50 nm in the dense layer is calculated from the ratio of the number of pores having a pore size of more than 50 nm to the total number of pores in one visual field of the dense layer, as the average in each visual field based on the following formula (1).

Total number of pores of more than 50 nm in one visual field of dense layer/Total number of pores in the same visual field × 100 ... (1)

The ratio of a pore having a pore size of 10 nm or smaller in the dense layer is calculated from the ratio of the number of pores having a pore size of 10 nm or smaller to the total number of pores in one visual field of the dense layer, as the average in each visual field based on the following expression (2).

Total number of pores of 10 nm or smaller in one visual field of dense layer/Total number of pores in the same visual field × 100 ... (2)

In the porous hollow fiber membrane of the present embodiment, when 1.5% by mass immunoglobulin is filtered at a constant pressure of 2.0 bar from the inner surface of the hollow fiber to the outer surface thereof, the ratio (F₆₀/F₁₀) of the immunoglobulin flux F₆₀ from a point of a lapse of 50 minutes to a point of a lapse of 60 minutes after the start of filtration to the immunoglobulin flux F₁₀ from the start of filtration to a point of a lapse of 10 minutes after the start of filtration is preferably 0.60 or more, more preferably 0.70 or more, further preferably 0.80 or more.

In the present embodiment, filtration conditions for evaluating a suppression of the lowering of the flux with time are as follows.

Protein filtration conditions are difficult to generally determine because they are varied depending on the application of filtration, the type and concentration of protein, and the like. However, immunoglobulin, which is a main substance to be filtered in use of a filtration membrane, is appropriately selected as a protein to be filtered.

The concentration of immunoglobulin solution has tended to be increased in recent years for the purpose of an enhancement in production efficiency, and therefore the concentration of immunoglobulin is appropriately 1.5% by mass.

In addition, filtration at high pressure increases a flux to enable to recover immunoglobulin at a high efficiency. However, when the pressure is too much high, sealability is difficult to retain at a connection between a filter and piping, and therefore the filtration pressure is appropriately 2.0 bar.

The parvovirus clearance as a filtration membrane in an application to protein solution treatment is preferably 4 or more, more preferably 5 or more as LRV. Porcine parvovirus (PPV) is preferable as parvovirus because of being close to a real liquid and simple in operations.

In the present embodiment, the ratio (F₆₀/F₁₀) of the immunoglobulin flux F₆₀ from a point of a lapse of 50 minutes to a point of a lapse of 60 minutes after the start of filtration to the immunoglobulin flux F₁₀ from the start of filtration to a point of a lapse of 10 minutes after the start of filtration, and the porcine parvovirus clearance can be measured by methods described in Examples.

In the present embodiment, the method for producing the porous hollow fiber membrane is a wet phase separation method, and is exemplified as follows.

A dope is obtained by mixing and dissolving the polysulfone-based polymer, the hydrophilic polymer, a solvent and a non-solvent, and degassing the resultant mixture; the dope and a bore liquid are ejected together through the annular portion and the center portion, respectively, of a double tube nozzle (spinneret) at the same time; and the resulting spin fiber is introduced through an air gap portion into a coagulation bath to form a hollow fiber. The obtained membrane is wound after washing with water, is subjected to removal of liquid in the hollow portion and then heat treatment, and is dried.

Any solvent can be widely used as the solvent for use in the dope as long as it is a good solvent to the polysulfone-based polymer and the hydrophilic polymer, and is compatible with the polysulfone-based polymer and the hydrophilic polymer. Examples thereof include N-methyl-2-pyrrolidone (NMP), N,N-dimethylformamide (DMF), N,N-dimethylacetamide (DMAc), dimethylsulfoxide and ε-caprolactam, amide-based solvents such as NMP. DMF and DMAc are preferable, and NMP is more preferable.

A non-solvent is preferably added to the dope. Examples of the non-solvent for use in the dope include glycerin, water and a diol compound, and a diol compound is preferable.

The diol compound is a compound having a hydroxyl group at each of both ends of its molecule, and as the diol compound, a compound represented by the following formula 3 and having an ethylene glycol structure in which the number of repeating units, n, is one or more is preferable.

Examples of the diol compound include diethylene glycol (DEG), triethylene glycol (TriEG), tetraethylene glycol (TetraEG) and polyethylene glycol, DEG, TriEG and TetraEG are preferable, and TriEG is more preferable.

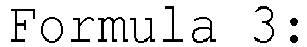

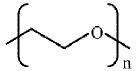

Although no exact mechanism is clear, it is considered as follows: the non-solvent is added to the dope, thereby suppressing the diffusion rate of the non-solvent in a coagulation liquid, thereby suppressing coagulation and easily controlling a preferable structure as the porous hollow fiber membrane to thereby suitably form a desired structure.

While the amounts of the solvent and the non-solvent mixed in the dope are preferably almost the same, the mass ratio of the solvent/non-solvent in the dope is preferably 35/65 to 65/35. A mass ratio of the solvent to the non-solvent of 65/35 or less allows coagulation to progress at a proper rate to thereby hardly cause an excessively large pore size, easily providing a porous hollow fiber membrane having a membrane structure preferable in an application to protein solution treatment. When the non-solvent satisfies a preferable mass ratio of 35/65 or more, coagulation does not progress so fast that an excessively small pore size is hardly caused and also any macrovoid resulting in a structure defect is hardly caused.

The concentration of the polysulfone-based polymer in the dope is preferably 15 to 35% by mass, more preferably 20 to 30% by mass.

When the concentration of the polysulfone-based polymer in the dope is 15% by mass or more, a proper membrane strength can be provided, porosity is not so high, and also sufficient virus removability can be exhibited without any excessive increase in porosity. When the concentration of the polysulfone-based polymer in the dope is 35% by mass or less, porosity is not so low, which enables permeation performance to be maintained and also enables the virus capture capacity of the membrane to be kept high.

The concentration of the hydrophilic polymer in the dope is preferably 5 to 12% by mass.

When the concentration of the hydrophilic polymer in the dope is 5% by mass or more, the resulting membrane can be sufficiently hydrophilicidized. Such a concentration is preferable because, even when the membrane is used for filtration of a protein solution, protein hardly adsorbs to the membrane to thereby hardly decrease a flux. When the concentration of the hydrophilic polymer in the dope is 12% by mass or less, the thickness of the hydrophilic polymer of the pore surface in the resulting membrane is not so high and the pore size is not excessively small. Such a concentration is also preferable in view of preventing fiber adhesion, where hollow fibers adhere to each other after drying.

The dope is obtained by dissolving the polysulfone-based polymer, the hydrophilic polymer, the solvent and the non-solvent with stirring at a constant temperature. The temperature here is preferably 30 to 80°C higher than ordinary temperature. A tertiary or less nitrogen-containing compound (such as NMP and vinylpyrrolidone) is oxidized in air and further easily progresses in oxidation when warmed, and therefore the dope is preferably prepared in an inert gas atmosphere. Examples of such an inert gas include nitrogen and argon, and nitrogen is preferable in terms of production cost.

If bubbles are present in the dope, large bubbles cause fiber breakage to occur during spinning and small bubbles cause any macrovoid to be formed after membrane formation to generate the structure defect of the membrane. Therefore degassing is preferably performed.

The degassing step can be performed as follows.

The content of a tank where the dope completely dissolved is placed is warmed to 50°C, depressurized to 2 kPa, and left to stand for 1 hour or more. Such operations are preferably repeated seven times or more. The pressure in degassing is preferably higher than the boiling point of the solvent. In order to increase degassing efficiency, the dope may be stirred during degassing.

Foreign substances in the dope are preferably removed before the dope is ejected through the spinneret. Large foreign substances cause fiber breakage to occur during spinning and small foreign substances cause the structure defect of the membrane to be generated. A raw material with few foreign substances can be used to thereby reduce the risk of contamination of foreign substances.

In order to eliminate contamination of foreign substances from the packing and the like of a dope tank, a filter may be disposed before the dope is ejected through the spinneret, a filter different in pore size may be disposed, or such filters different in pore size may be disposed at multistage. Specifically, a mesh filter having a pore size of 30 µm and a mesh filter having a pore size of 10 µm may be disposed closer to the dope tank in this order.

The same components as the components for use in the dope and the coagulation liquid are preferably used in the bore liquid for use in membrane formation. For example, when NMP/TriEG and NMP/TriEG-water are used as the solvent/non-solvent of the dope and the solvent/non-solvent of the coagulation liquid, respectively, the bore liquid is preferably configured from NMP-TriEG-water.

When the amount of the solvent in the bore liquid is larger, the effects of delaying progression of coagulation and allowing membrane structure formation to slowly progress are exerted. When the amount of the non-solvent in the bore liquid is larger, the effects of delaying diffusion of the solution, delaying progression of coagulation and allowing membrane structure formation to slowly progress are exerted by the thickening effect. When the amount of water in the bore liquid is larger, the effect of promoting progression of coagulation is exerted.

In order to allow coagulation not to so rapidly or so slowly progress, but to properly progress; to control the membrane structure; and to provide a porous hollow fiber membrane having a preferable membrane structure as a filtration membrane, the solvent and the non-solvent as organic components in the bore liquid are preferably used in almost the same amounts, the ratio of the solvent/non-solvent as organic components in the bore liquid is preferably 35/65 to 65/35 in a weight ratio and the ratio of the organic components/water is preferably 70/30 to 100/0 in a mass ratio.

The spinneret temperature is preferably 40°C or more in view of properly promoting progression of coagulation and preventing the pore size from being excessively larger, and the spinneret temperature is preferably 60°C or less in view of preventing progression of coagulation from being so fast that the pore size is excessively small.

The dope is ejected through the spinneret, thereafter passes through the air gap portion, and is introduced into the coagulation bath. The retention time in the air gap portionis preferably 0.01 seconds or more in view of allowing for sufficient coagulation before introduction into the coagulation bath to prevent the pore size from being excessively small, and is preferably 0.75 seconds or less in view of not allowing coagulation to excessively progress before introduction into the coagulation bath, to enable the membrane structure to be precisely controlled in the coagulation bath. It is more preferably 0.05 to 0.4 seconds.

The draft ratio is preferably 1.1 to 6, more preferably 1.1 to 4, in order to control drawing to the hollow fiber membrane in the spinning step. The draft ratio means the ratio of the taking-over rate to the linear velocity of the dope ejected through the spinneret.

A higher draft ratio means a higher draw ratio after ejection through the spinneret.

In the case where the hollow fiber membrane is formed by a wet phase separation method, a general membrane structure is determined when the dope passes through the air gap portion and exits from the coagulation bath. The interior of the membrane is configured from a solid portion formed from polymer chains entangled and an imaginary portion serving as a pore portion where no polymer is present. Although no exact mechanism is clear, a possible mechanism is as follows: when the hollow fiber membrane is excessively drawn before completion of coagulation, in other words, excessively drawn before polymer chains are entangled, entanglement of the polymer chains is torn and pore portions are linked to thereby form an excessively large pore and be divided to a pore portion, thereby forming an excessively small pore. An excessively large pore causes viruses to be leaked, and an excessively small pore causes clogging to occur. Even a slight structure defect is detrimental to the filtration membrane, and therefore the draft ratio is preferably as low as possible.

The dope passes through the filter and the spinneret, is properly coagulated in the air gap portion, and thereafter is introduced into the coagulation liquid. If a membrane where coagulation is not completed is introduced into a general coagulation bath where the coagulation liquid is merely placed, bath resistivity and friction resistivity due to contact with a roll in the coagulation bath allow a drawing force to act on the membrane. In the present embodiment, although no exact mechanism is clear, the coagulation liquid can be allowed to flow in parallel with the spinning direction not to thereby cause any drawing to be applied to the membrane where coagulation is not completed, as far as possible, thereby inhibiting an excessively small pore and an excessively large pore from being formed in the dense layer. In addition, it is considered that the coagulation liquid is allowed to flow in parallel with the spinning direction and therefore liquid exchange at an interface closer to the outer surface of the hollow fiber is moderately performed, thereby allowing coagulation to more moderately progress than a case where a general coagulation bath is used, to thereby make the dense layer thicker. In addition, in general, coagulation moderately progresses to make the pore size distribution broader, and therefore coagulation moderately progresses to lead to the presence of a large pore in the dense layer.

The same components as the components for use in the dope and the bore liquid are preferably used in the coagulation liquid for use in membrane formation.

An organic component has the effect of delaying coagulation and water has the effect of promoting coagulation. Therefore, the composition of the coagulation liquid is as follows: the ratio of the solvent/non-solvent as organic components is preferably 35/65 to 65/35 in a mass ratio, and the ratio of the organic components/water is preferably 90/10 to 50/50, more preferably 60/40 to 40/60.

While the flow velocity of the coagulation liquid may be set so as not to cause the coagulation liquid to be excessively drawn to the hollow fiber, the ratio of the linear velocity of the coagulation liquid to the linear velocity of spinning is preferably 0.7 to 1.3 in view of preventing any structure defect from occurring due to drawing of the membrane.

The coagulation bath temperature is preferably 30 to 60°C in view of control of the pore size.

The spinning rate is preferably set to a low rate. The spinning rate can be low to thereby thicken a boundary film formed at an interface between the outer surface of the hollow fiber and the coagulation liquid, moderately performing liquid exchange at the interface. The spinning rate is set to a low rate to lead to thickening of the dense layer. A preferable lower limit of the spinning rate is set so that production efficiency is ensured. Specifically, the lower limit is preferably 4 to 10 m/min.

The hollow fiber membrane pulled up from the coagulation bath is washed with warm water.

In the water washing step, the solvent and a hydrophilic polymer not immobilized in the membrane are preferably certainly removed. If the hollow fiber membrane is dried with including the solvent, the solvent can be condensed in the membrane during drying and the polysulfone-based polymer can be dissolved or swollen to thereby change the membrane structure. The hydrophilic polymer not immobilized in the membrane can remain to cause a pore to be blocked, resulting in degradation of permeability of the membrane.

In order to increase the diffusion rates of the solvent/non-solvent and the hydrophilic polymer not immobilized in the membrane, which are to be removed, and to increase water washing efficiency, the temperature of warm water is preferably 50°C or more. In the water washing step, a Nelson roller is preferably used.

In order to sufficiently perform water washing, the retention time of the hollow fiber membrane in a water washing bath is preferably 80 to 300 seconds. While the water washing step for the purpose of removal of any unnecessary component is more preferably performed for a longer time, the water washing step is properly performed for a time of 300 seconds or less in terms of production efficiency.

The hollow fiber membrane pulled up from the water washing bath is wound to a winding frame with a winder. If the hollow fiber membrane is here wound in air, the membrane is gradually dried and is slightly shrunk. The degree of shrinkage of the membrane differs between the initial stage and the later stage of winding-up and the membrane structure thus differs therebetween to thereby cause a hollow fiber membrane obtained in a production step to have ununiformity. In order to provide a uniform membrane, the hollow fiber membrane is preferably wound in water.

The hollow fiber membrane wound to a winding frame, from which both ends are cut out, is then bundled, and grasped to a support so as not to be loosed. The hollow fiber membrane grasped is then immersed in hot water and washed.

A clouded liquid where nanometer to micrometer-sized fine particles of the polysulfone-based polymer are floating remains in the hollow portion of the hollow fiber membrane wound to a winding frame. If the hollow fiber membrane is dried with the clouded liquid being not removed, the fine particles of the polysulfone-based polymer block pores of the hollow fiber membrane to result in degradation of membrane performance in some cases, and therefore the clouded liquid in the hollow portion is preferably removed.

The hollow fiber membrane is also washed from the inner surface thereof in the hot water treatment step, and therefore a hydrophilic polymer and the like not completely removed in the water washing step and not immobilized in the membrane are efficiently removed. The temperature of hot water is preferably 50 to 100°C. The temperature of hot water is preferably 50°C or more because washing efficiency can be increased.

The washing time is preferably 30 to 120 minutes. Hot water is preferably exchanged several times during washing.

In the present embodiment, the hollow fiber membrane wound up is preferably treated with hot water at high pressure. Specifically, preferably, the hollow fiber membrane is placed in a high-pressure steam sterilizer in the state of being completely immersed in water, and retained at 120°C or more for 2 to 6 hours. Although no exact mechanism is known, it is considered that such a high-pressure hot water treatment not only allows the solvent/non-solvent slightly remaining in the hollow fiber membrane and a hydrophilic polymer not adhering to the membrane to be completely removed, but also allows the state where the polysulfone-based polymer and the hydrophilic polymer are present to be optimized and allows a preferable structure for the filtration membrane to be optimized. The treatment time is preferably 6 hours or less in terms of production efficiency.

The porous hollow fiber membrane of the present embodiment is obtained by drying by air, under reduced pressure, by hot air, and the like. Without any particular limitation, the hollow fiber membrane is preferably dried with both ends thereof being fixed so that the membrane is not shrunk during drying.

### Examples

Hereinafter, the present embodiment is described in detail with respect to Examples, but the present invention is not intended to be limited to the following Examples. Measurement methods shown in Examples are as follows.

### (1) Measurements of inner diameter and membrane thickness

The inner diameter of the porous hollow fiber membrane was determined by photographing the torn vertical section of the membrane by a stereoscopic microscope.

The outer diameter was determined in the same manner as in the inner diameter, and the membrane thickness was determined from (outer diameter - inner diameter)/2.

The membrane area was calculated from the inner diameter and the effective length of the hollow fiber membrane.

### (2) Measurement of pure water permeation rate

A filter assembled so that the porous hollow fiber membrane had a number of hollow fibers of 4 and an effective length of 8 cm was prepared, the amount of filtration of pure water at 25°C using the filter by dead-end type filtration at a constant pressure of 1.0 bar was measured, and the amount of permeation of water was measured from the filtration time to calculate the pure water permeation rate from the effective membrane area.

### (3) Filtration test of immunoglobulin

A filter assembled so that the porous hollow fiber membrane had a number of hollow fibers of 4 and an effective length of 8 cm was subjected to a high-pressure sterilization treatment at 122°C for 60 minutes. A solution was prepared using donated blood Venoglobulin IH 5% intravenous (2.5 g/50 mL) commercially available from Mitsubishi Tanabe Pharma Corporation so that the solution had an immunoglobulin concentration of 1.5% by mass, a sodium chloride concentration of 0.1 M and a pH of 4.5. The solution prepared was filtered by a dead-end system at a constant pressure of 2.0 bar for 60 minutes.

The filtrate was here recovered at an interval of 10 minutes, and the ratio of the amount of the filtrate recovered between 50 minutes and 60 minutes to the amount of the filtrate recovered between 0 minutes and 10 minutes was defined as F₆₀/F₁₀.

### (4) Measurement of porcine parvovirus clearance

A solution where a 0.5% by vol PPV solution was spiked in the solution prepared in (3) Filtration test of immunoglobulin was used as the solution filtered. The same filtration test as in (3) Filtration test of immunoglobulin was performed.

The Titer (TCID₅₀ value) of the filtrate was measured by a virus assay. The virus clearance of PPV was calculated according to LRV = Log(TCID₅₀)/mL (solution filtered) - Log (TCID₅₀)/mL (filtrate).

### (5) Thickness of dense layer

The cross section of the hollow fiber was photographed with a scanning electron microscope (SEM) while the acceleration voltage was set to 1 kV, the photographing magnification was set to 50,000-magnification, and the visual field was horizontally set at an arbitrary portion of the cross section of the hollow fiber in the membrane thickness direction. After photographing at one visual field set, the visual field to be photographed is horizontally moved in the membrane thickness direction, and the next visual field was photographed. Such photographing operations were repeated to take the photograph of the cross section of the membrane without any space, and the photographs obtained were combined to thereby provide one photograph of the cross section of the membrane. With respect to the photograph of the cross section, the average pore size was calculated in an area of (2 µm in the circumferential direction of the membrane) × (1 µm from the outer surface towards the inner surface).

The calculation method of the average pore size was according to calculation by a method using image analysis. The pore portion and the solid portion were distinguished based on the brightness by use of Image-pro plus manufactured by MediaCybernetics Inc. A portion which could not be distinguished and a noise were corrected by a free hand tool, and an edge portion serving as a profile of the pore portion and a porous structure observed at the back of the pore portion were distinguished as the pore portion. After binarization processing, the area value of one pore was assumed as the area of a true circle and the pore diameter was calculated to two significant figures. Such operations were performed with respect to each pore, and the average pore size was sequentially calculated with respect to each area of 1 µm × 2 µm. A pore portion cut at the end of the visual field was also counted as such an area.

A visual field where the average pore size was 50 nm or smaller was defined as the dense layer, and the thickness of the dense layer was defined as "the number of images exhibiting an average pore size of 50 nm or smaller × 1 µm".

### (6) Ratio of pore having a pore size of more than 50 nm and ratio of pore having a pore size of 10 nm or smaller in dense layer

The ratio of a pore having a pore size of more than 50 nm in the dense layer was calculated from the ratio of the number of pores having a pore size of more than 50 nm to the total number of pores in one visual field of the dense layer, as the average in each visual field based on the following formula (1).

Total number of pores of more than 50 nm in one visual field of dense layer/Total number of pores in the same visual field × 100 ... (1)

The ratio of a pore having a pore size of 10 nm or smaller in the dense layer was calculated from the ratio of the number of pores having a pore size of 10 nm or smaller to the total number of pores in one visual field of the dense layer, as the average in each visual field based on the following expression (2).

Total number of pores of 10 nm or smaller in one visual field of dense layer/Total number of pores in the same visual field × 100 ... (2)

### (Example 1)

A solution obtained by mixing 26 parts by mass of a polyethersulfone (PES) (Ultrason (trade name) E6020P manufactured by BASF SE), 10 parts by mass of a copolymer of vinylpyrrolidone and vinyl acetate (Luviskol (registered trademark) VA64 manufactured by BASF SE, hereinafter, designated as "VA64"), 32 parts by mass of NMP (manufactured by Kishida Chemical Co., Ltd.) and 32 parts by mass of TriEG (manufactured by Kanto Kagaku) at 50°C and thereafter repeating degassing under reduced pressure seven times was defined as a dope.

The dope was ejected through the annular portion of a double tube nozzle, and a mixed liquid of 42.8 parts by mass of NMP, 52.2 parts by mass of TriEG and 5 parts by mass of water was ejected as a bore liquid through the center portion thereof. The temperatures of the dope and the bore liquid ejected through the double tube nozzle were here regulated so as to be 50°C. The dope and the bore liquid ejected passed through an air gap portion and were travelled by 2 m in a tube having a diameter of 1.0 cm where a coagulation liquid including 38.3 parts by mass of NMP, 46.7 parts by mass of TriEG and 15 parts by mass of water at 20°C flowed at a flow velocity of 390 mL/min. The hollow fiber membrane pulled out from the coagulation bath was travelled in a water washing tank set to 55°C with a Nelson roll, and wound up in water. The spinning rate was 5 m/min and the draft ratio was 2.

The hollow fiber membrane wound up, from which both ends were cut out, was then bundled, grasped to a support so as not to be loosed, immersed in hot water at 80°C, and washed for 60 minutes. The hollow fiber membrane washed was treated with hot water at high pressure in conditions of 128°C and 3 hours, and thereafter dried in vacuum to provide a porous hollow fiber membrane.

### (Example 2)

A porous hollow fiber membrane was obtained in the same manner as in Example 1 except that the composition of the dope was changed to 24 parts by mass of PES, 12 parts by mass of VA64, 30.4 parts by mass of NMP and 33.6 parts by mass of TriEG, the composition of the bore liquid was changed to 52.8 parts by mass of NMP, 42.2 parts by mass of TriEG and 5 parts by mass of water, the coagulation liquid temperature was changed to 30°C, and the composition of the coagulation liquid was changed to 38.9 parts by mass of NMP, 31.1 parts by mass of TriEG and 30 parts by mass of water.

### (Example 3)

A porous hollow fiber membrane was obtained in the same manner as in Example 1 except that the composition of the coagulation liquid was changed to 40.5 parts by mass of NMP, 49.5 parts by mass of TriEG and 10 parts by mass of water.

### (Example 4)

A porous hollow fiber membrane was obtained in the same manner as in Example 2 except that the composition of the coagulation liquid was changed to 41.7 parts by mass of NMP, 33.3 parts by mass of TriEG and 25 parts by mass of water, the coagulation liquid temperature was changed to 35°C, and the draft ratio was changed to 1.5.

### (Example 5)

A porous hollow fiber membrane was obtained in the same manner as in Example 2 except that the coagulation liquid temperature was changed to 20°C.

The results of measurements (1) to (6) of the respective porous hollow fiber membrane obtained in Examples 1 to 5 are shown in Table 1.

All the porous hollow fiber membranes obtained in Examples 1 to 5 exhibited sufficient performance for removing a virus and the like included in the solution, suppressed the lowering of flux with time during filtration of the protein solution, and had excellent permeability to a useful component.

### (Comparative Example 1)

A hollow fiber membrane was obtained in the same manner as in Example 1 except that the composition of the coagulation liquid was changed to 40.5 parts by mass of NMP, 49.5 parts by mass of TriEG and 10 parts by mass of water, and a coagulation bath where no coagulation liquid flowed was used.

The coagulation bath where no coagulation liquid flowed was used to thereby cause the dense layer to be thinned, making impossible to suppress the lowering of flux with time during filtration of the protein solution. In addition, virus removability was also degraded.

### (Comparative Example 2)

A hollow fiber membrane was obtained in the same manner as in Example 1 except that the spinning rate was changed to 20 m/min and the draft ratio was changed to 10.

The spinning rate was increased and the draft ratio was increased to thereby cause the dense layer to be thinned, making impossible to suppress the lowering of flux with time during filtration of the protein solution. In addition, virus removability was also degraded.

### (Comparative Example 3)

A hollow fiber membrane was obtained in the same manner as in Example 1 except that the composition of the coagulation liquid was change to 100 parts by mass of water, the coagulation liquid temperature was change to 45°C, and a coagulation bath where no coagulation liquid flowed was used.

The composition of the coagulation liquid was changed to 100 parts by mass of water to thereby cause the dense layer to be thinned and cause clogging to be remarkable, making impossible to filter protein for 60 minutes.

### (Comparative Example 4)

A hollow fiber membrane was obtained in the same manner as in Example 1 except that the composition of the coagulation liquid was changed to 30 parts by mass of NMP, 60 parts by mass of TriEG and 10 parts by mass of water, the coagulation liquid temperature was changed to 15°C, and the draft ratio was changed to 1.5.

The dense layer was so thick that excellent protein permeability was not realized.

**[Table 1]**

| | Example 1 | Example 2 | Example 3 | Example 4 | Example 5 | Comparative Example 1 | Comparative Example 2 | Comparative Example 3 | Comparative Example 4 |
|---|---|---|---|---|---|---|---|---|---|
| Thickness of dense layer (µm) | 21 | 10 | 28 | 13 | 13 | 4 | 6 | 1 | 33 |
| Inner diameter (µm) | 198 | 200 | 201 | 200 | 199 | 199 | 198 | 196 | 205 |
| Membrane thickness (µm) | 58 | 60 | 61 | 59 | 61 | 59 | 56 | 55 | 62 |
| Ratio of pore having a pore diameter of more than 50 nm in dense layer (%) | 32 | 29 | 36 | 35 | 26 | 26 | 27 | 22 | 41 |
| Ratio of pore having a pore diameter of 10 nm or smaller in dense layer (%) | 12 | 14 | 10 | 10 | 19 | 18 | 26 | 33 | 9 |
| LRV | 5 | 4 | 5 | 4 | 5 | 3 | 3 | - | 3.5 |
| F₆₀/F₁₀ | 0.81 | 0.75 | 0.83 | 0.65 | 0.69 | 0.55 | 0.58 | - | 0.84 |
| Pure water permeation rate (L/(hr·m2·bar)) | 75 | 83 | 50 | 175 | 35 | 186 | 94 | 310 | 28 |

The present application is based on Japanese Patent Application (Japanese Patent Application No. 2015-7073) filed on January 16, 2015, the content of which is herein incorporated by reference.

### Industrial Applicability

The porous hollow fiber membrane of the present invention has industrial applicability because of being excellent in virus removal and protein permeability during filtration of a protein solution.

## Claims

1. A porous hollow fiber membrane comprising a polysulfone-based polymer and a hydrophilic polymer, and
having a dense layer in a section from an outer surface portion to a center region of a membrane thickness, a thickness of the dense layer being 10 to 30 µm, and a ratio of a pore having a pore size of more than 50 nm and a ratio of a pore having a pore size of 10 nm or smaller in the dense layer being 25 to 40% and 20% or less, respectively.

2. The porous hollow fiber membrane according to claim 1, wherein the polysulfone-based polymer is a polyethersulfone.

3. The porous hollow fiber membrane according to claim 1 or 2, wherein the hydrophilic polymer is a copolymer of vinylpyrrolidone and vinyl acetate.

4. The porous hollow fiber membrane according to any one of claims 1 to 3, wherein a pure water permeation rate is 40 to 180 L/(hr·m²·bar).

5. The porous hollow fiber membrane according to any one of claims 1 to 4, wherein, when 1.5% by mass immunoglobulin is filtered at a constant pressure of 2.0 bar from an inner surface of a hollow fiber to an outer surface thereof, a ratio (F₆₀/F₁₀) of immunoglobulin flux F₆₀ from a point of a lapse of 50 minutes to a point of a lapse of 60 minutes after the start of filtration to immunoglobulin flux F₁₀ from the start of filtration to a point of a lapse of 10 minutes after the start of filtration is 0.60 or more.

6. The porous hollow fiber membrane according to any one of claims 1 to 5, for use in removing a virus contaminated in a protein solution and recovery of protein.
